# EUROPEAN PATENT APPLICATION

(11) **EP 2 857 399 A1**
(43) Date of publication of application: **08.04.2015**
(21) Application number: 13187200.4
(22) Date of filing: 03.10.2013
(51) Int. Cl.: C07D 471/04, A61K 31/55, A61P 15/10

(54) **Compounds useful for the treatment of PDE5 inhibitor-poorly responsive erectile dysfunction**

(71) Applicant: GenKyoTex SA, 1228 Plan-les-Ouates (CH)
(72) Inventor: Meldrum, Eric, 4125 BASEL (CH); Page, Patrick, 74160 SAINT- JULIEN-EN-GENEVOIS (FR); Szyndralewiez, Cédric, 74350 ANDILLY (FR)
(74) Representative: reuteler & cie SA

(57) **Abstract**

The present invention is related to compounds that are able to restore responsiveness to PDE5 inhibitors for patients presenting a PDE5 inhibitor non-responsive or PDE5 inhibitor poorly responsive erectile dysfunction. The present invention is directed to compositions and methods for the restoration of responsiveness to PDE5 inhibitors, in particular for the restoration of responsiveness of the endothelial tissues of *corpus cavernosum.*

## Description

### Field of the Invention

The present invention relates to agents and combination of agents useful in the treatment and/or prevention of erectile dysfunction which is poorly responsive to the treatment by inhibitors of 5 phosphodiesterase (PDE5) enzyme such as Sildenafil.

### Background of the Invention

Erectile dysfunction (ED) is defined as the inability to attain/maintain penile erection sufficient for satisfactory sexual performance. The Massachusetts Male Aging Study established that the prevalence of ED increases with age as it affects up to the half of the male population between 40-70 years old (Lasker et al., 2010, Adv. Pharmacol. Sci.*).* Thus since the world's older population increases, it has been estimated that the prevalence of ED will reach about 322 million in 2025, indicating that there is a need of re-evaluating of current ED therapeutic strategies *(*Hatzimouratidis et al., 2008, Drugs, 68(2), 231-50*).* Further, it has been shown that erectile dysfunction rates increase as a function of vascular comorbidities such as hypertension, diabetes mellitus, obesity and atherosclerosis (*Lasker et al., 2010, supra)* but also dyslipidemia, smoking and depression (Kendirci et al.,2006, Current Opinion in Urology, 16:449-459)*.* According to the Canadian Diabetes Association (CDA), erectile dysfunction (ED) is common for men who have diabetes. Erectile dysfunction is often the first symptom that men may notice and the one that leads them to the doctor in the first place. Only after they have sought medical help for ED do they also receive a diagnosis of diabetes. Fifty percent of men with diabetes currently suffer from ED.

Penile erection is the end result of a chain of events that induce the smooth-muscle relaxation in the *corpus cavernosum* of the penis. Nitric Oxide (NO) is a key mediator in the initiation and maintenance of penile erection and is produced and released by the endothelium and the autonomic nerves of penile arteries and corpus cavemosum tissue. Once released, NO enters the smooth muscle cell and activates soluble guanylyl cyclase which promotes the formation of cyclic guanosine monophosphate (cGMP), which levels are mainly regulated by PDE5 enzyme, a cGMP specific hydrolyzing enzyme. PDE5 is present in high concentrations in the smooth-muscle of *corpora carvernosa* of the penis and in the smooth-muscle of the pudental arteries. Therefore, PDE5 inhibitors are the major initial pharmacotherapy strategy for ED among the various existing treatments (Davis et al., 2012, Current Perspectives on Pharmacotherapy Treatments for Erectile Dysfunction in Erective Dysfunction- Disease-Associated Mechanisms and Novel Insights into Therapy, Ed. Kenia Nunes, Intech)*.*

Sildenafil is a PDE5 inhibitor and first line therapy used in the treatment of erectile dysfunction of various etiologies. However, sildenafil treatment failure or low efficacy has been reported for some ED patients, in particular, in patients with diabetes and with severe neurological damage (e.g. due to radical prostatectomy), the efficacy of sildenafil drops to 56 and 45% of the patients treated, respectively (Rendell et al., 1999, JAMA, 281, 421-6*; Kenderci et al., 2006, supra*)*.* Furthermore, for type-2 diabetes mellitus (T2DM), in spite of an initial response to PDE5 inhibitor treatment, the effects are not sustainable over time and after 12 months of ED treatment, mean International Index of Erectile Dysfunction (IIEF) scores for T2DM patients reverted to baseline values (Hidalgo-Tamola et al., 2009, J. Sex Med., 6, 916-926*;* Penson et al., Diabetes Care, 2003, 26, 1093-9*.*

Therefore, patient populations with diabetes mellitus, severe neurological damage or severe vascular disease are considered as particularly challenging to treat with PDE5 inhibitor therapy. In addition, some non neglectable sub-populations of PDE5 inhibitor responding patients such as those listed in *Kendirci et al., 2006, supra* are not responding or poorly responding to PDE5 inhibitor treatments. Therefore, due to the importance of cumulated-non responding or poorly responding population, it would be highly desirable to develop new treatment strategy for the treatment of PDE5 inhibitor-non responding or poorly responding erectile dysfunction.

### Summary of the Invention

The present invention is directed towards the unexpected findings that compounds of the invention are able to restore responsiveness to PDE5 inhibitors in PDE5 inhibitor non-responsive or PDE5 inhibitor poorly responsive erectile dysfunction context. The present invention is directed to compositions and methods for the restoration of responsiveness to PDE5 inhibitors, in particular for the restoration of responsiveness of the endothelial tissues of *corpus cavernosum.* Notably, the invention is related to new molecules useful in the prevention and/or treatment of PDE5 inhibitor non-responsive or poorly responsive erectile dysfunction.

A first aspect of the invention provides a compound according to Formula (I) for the treatment and/or prophylaxis of PDE5 inhibitor non-responsive or poorly responsive erectile dysfunction.

A second aspect of the invention provides a use of one or more compound according to Formula (I) for the preparation of a pharmaceutical composition for the treatment and/or prophylaxis of PDE5 inhibitor non-responsive or poorly responsive erectile dysfunction. A third aspect of the invention provides new compound according to Formula (I), as well as tautomers, geometrical isomers, optically active forms, and pharmaceutically acceptable salts thereof.

A fourth aspect of the invention relates to new compounds according to Formula (I), as well as tautomers, geometrical isomers, optically active forms, and pharmaceutically acceptable salts thereof for use as a medicament.

A fifth aspect of the invention relates to a pharmaceutical composition containing at least one compound according to Formula (I) according to the invention, as well as tautomers, geometrical isomers, optically active forms and pharmaceutically acceptable salts thereof and a pharmaceutically acceptable carrier, diluent or excipient thereof.

A sixth aspect of the invention relates to a pharmaceutical composition containing at least one compound according to Formula (I) combined with at least one PDE5 inhibitor and at least one pharmaceutically acceptable carrier.

A seventh aspect of the invention relates to a method for treating a patient suffering from PDE5 inhibitor non-responsive or poorly responsive erectile dysfunction, comprising administering an effective amount of one or more compound according to Formula (I), in a patient in need thereof.

An eighth aspect of the invention relates to a method for restoring or increasing responsiveness to PDE5 inhibitor treatment, in particular restoring or increasing responsiveness of endothelial tissues of *corpus cavernosum* to a PDE5 inhibitor in a patient comprising administering an effective amount of one or more compound according to Formula (I) or a pharmaceutical formulation thereof in a patient in need thereof.

A ninth aspect of the invention relates to a pharmaceutical composition containing at least one compound according to Formula (I) combined or not with at least one PDE5 inhibitor and at least one pharmaceutically acceptable carrier

Other features and advantages of the invention will be apparent from the following detailed description.

### Description of the figures

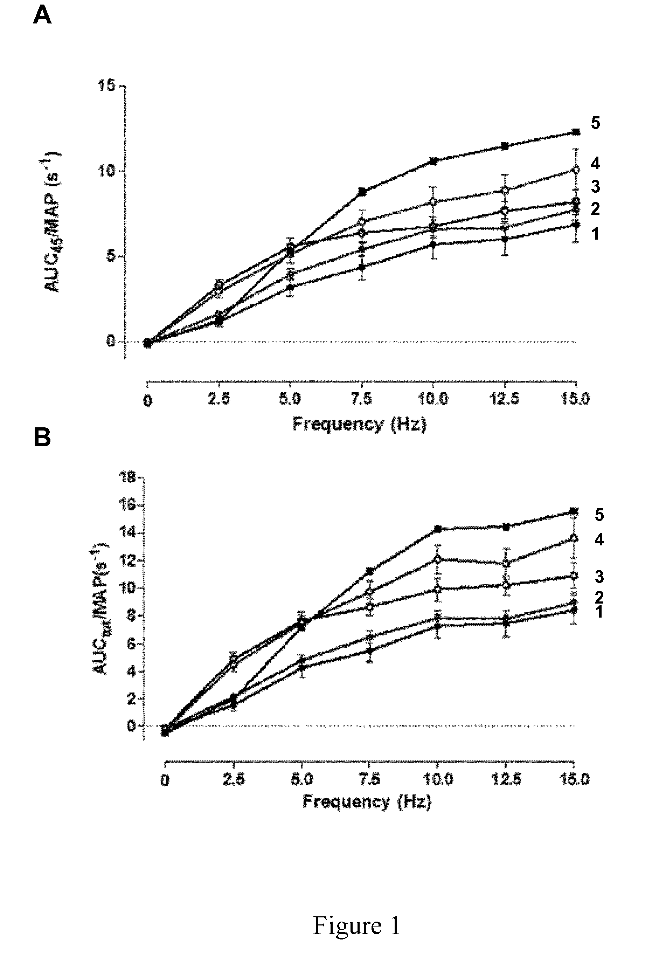

**Figure 1** shows the effect of treatment with a compound of the invention (compound 5) at 30 mg/kg on erectile response on diabetic rats as described in Example 1 comparing non-treated diabetic animals (1), diabetic animals treated chronically with compound 5 alone (2), diabetic animals treated acutely with sildenafil alone (3), diabetic animals treated with chronically with compound 5 and then acutely sildenafil (4) and non-treated wild-type animals (5) represented by **A:** the area under the curve during the first 45 seconds after the beginning of the electric stimulation (AUC₄₅/MAP) versus frequency; **B:** the area under the curve during the entire erectile response measured from the beginning of the electric stimulation until the end of the erectile response and determined using the ICP level in the flaccid state before the onset of the stimulation (AUCₜₒₜ/MAP) versus frequency of the stimulation.

### Detailed Description of the invention

The following paragraphs provide definitions of the various chemical moieties that make up the compounds according to the invention and are intended to apply uniformly through-out the specification and claims, unless an otherwise expressly set out definition provides a broader definition.

"PDE5 inhibitor" as used herein refers to any substances that are able to totally or partially inhibit, block, attenuate, or interfere with PDE5, or with any pathway elicited, either directly or indirectly, by PDE5. For example, PDE5 inhibitors include substances as described in Yuan, 2013, Eur. Urol., 63(5), 902-12*.* In particular, a PDE5 inhibitor according to the invention includes sildenafil, sildenafil citrate, udenafil, avanafil, lodenafil, tadalafil, mirodenafil and vardenafil. According to a particular embodiment, a PDE5 inhibitor according to the invention is sildenafil or sildenafil citrate.

"Non-responsiveness" or "poor responsiveness" to PDE5 inhibitor treatment is defined as the inability to attain or maintain an erection for satisfactory sexual intercourse or sexual activity following optimal dosing of the PDE5 inhibitor with adequate sexual stimulation, satisfactory sexual intercourse or sexual activity meaning an erection sufficient for penetration and/or lasting long enough to complete intercourse as a success.

The term "PDE5 inhibitor non-responsive or poorly responsive erectile dysfunction" includes erectile dysfunction symptoms which cannot be alleviated in a satisfactory manner through the treatment of a PDE5 inhibitor. PDE5 inhibitor non-responsive or poorly responsive erectile dysfunction includes sildenafil non-responsive or poorly responsive erectile dysfunction.

The term "efficacy" of a treatment or method according to the invention can be measured based on changes in the course of disease or condition in response to a use or a method according to the invention. For example, the efficacy of a treatment or method according to the invention can be measured by the assessment of its effectiveness in the amelioration or eradicating of various parameters of erectile dysfunction which are listed in validated questionnaires such as those from the International Index of Erectile Function (IIEF) (Rosen et al., 2002, Int. J. Impot. Res., 14(4):226-244)*.* Additional efficacy outcome instruments include a number of patient diaries and questionnaires such as the Sexual Encounter Profile and Global Assessment Question. These subjective patient-reported efficacy outcomes provide clinically meaningful information on erectile function. Objective measures of erectile function can also be used. For instance, the Rigiscan® methodology (ambulatory rigidity and tumescence scanner) which measures penis radial rigidity is widely used (Giesbers and al., New methods in the diagnosis of impotence: RigiScan® penile tumescence and rigidity monitoring and diagnostic papaverine hydrochloride injection, World Journal of Urology, 198 7, Volume 5, Issue 3, pp 173-176).

The term "effective amount" as used herein refers to an amount of at least one compound of Formula (I) of the invention or a pharmaceutical formulation thereof according to the invention that elicits a detectable amelioration/restoration of *corpus cavernosum* endothelium responsiveness to PDE5 treatment.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions.

The term "subject" as used herein refers to mammals. For examples, mammals contemplated by the present invention include human, primates, domesticated animals such as cattle, sheep, pigs, horses and the like as well as rodents.

The term "alkyl" when used alone or in combination with other terms, comprises a straight chain or branched C₁-C₂₀ alkyl which refers to monovalent alkyl groups having 1 to 20 carbon atoms. This term is exemplified by groups such as methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethylpropyl, n-hexyl, 2-methylpentyl, 3-methylpentyl, 4-methylpentyl, n-heptyl, 2-methylhexyl, 3-methylhexyl, 4-methylhexyl, 5-methylhexyl, n-heptyl, n-octyl, n-nonyl, n-decyl, tetrahydrogeranyl, n-dodecyl, n-tridecyl, n-tetradecyl, n-pentadecyl, n-hexadecyl, n-octadecyl, n-nonadecyl, and n-eicosanyl and the like. Preferably, these include C₁-C₉ alkyl, more preferably C₁-C₆ alkyl, especially preferably C₁-C₄ alkyl, which, by analogy, refer respectively to monovalent alkyl groups having 1 to 9 carbon atoms, monovalent alkyl groups having 1 to 6 carbon atoms and monovalent alkyl groups having 1 to 4 carbon atoms. Particularly, those include C₁-C₆ alkyl.

The term "aryl" refers to an unsaturated aromatic carbocyclic group of from 6 to 14 carbon atoms having a single ring (*e.g.,* phenyl) or multiple condensed rings (*e.g.,* indenyl, naphthyl). Aryl include phenyl, naphthyl, anthryl, phenanthrenyl and the like. The term "aryl C₁-C₆ alkyl" refers to C₁-C₆ alkyl groups having an aryl substituent, including 3-phenylpropanyl, benzyl and the like.

The term "heteroaryl" refers to a monocyclic heteroaromatic, or a bicyclic or a tricyclic fused-ring heteroaromatic group. Particular examples of heteroaromatic groups include optionally substituted pyridyl, pyrrolyl, pyrimidinyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadia-zolyl, 1,2,5-oxadiazolyl, 1,3,4-oxadiazolyl,1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxa-zolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

The term "heteroaryl C₁-C₆ alkyl" refers to C₁-C₆ alkyl groups having a heteroaryl substituent, including furyl methyl and the like.

The term "alkoxy C₁-C₆ alkyl" refers to C₁-C₆ alkyl groups having an alkoxy substituent, including methoxyethyl and the like.

The term "amino" refers to the group -NRR' where R and R' are independently H and any of the following groups which can be optionally substituted: "C₁-C₆ alkyl", "aryl", "heteroaryl", "C₁-C₆ alkyl aryl", "C₁-C₆ alkyl aryl C₁-C₆ alkyl", "C₁-C₆ alkyl heteroaryl," "heteroaryl C₁-C₆ alkyl", "cycloalkyl," or "heterocycloalkyl," and where R and R', together with the nitrogen atom to which they are attached, can optionally form a 3-8-membered heterocycloalkyl ring.

The term "amino alkyl" refers to alkyl groups having an amino substituent, including 2-(1-pyrrolidinyl)ethyl and the like.

Unless otherwise constrained by the definition of the individual substituent, the term "substituted" refers to groups substituted with from 1 to 5 substituents selected from the group consisting of "C₁-C₆ alkyl," "C₂-C₆ alkenyl," "C₂-C₆ alkynyl," "C₃-C₈-cycloalkyl," "heterocycloalkyl," "C₁-C₆ alkyl aryl," "C₁-C₆ alkyl heteroaryl," "C₁-C₆ alkyl cycloalkyl," "C₁-C₆ alkyl heterocycloalkyl," "amino," "aminosulfonyl," "ammonium," "alkoxy," "acyl", "acyl amino," "amino carbonyl," "aryl," "heteroaryl," "sulfinyl," "sulfonyl," "sulphonamide", "alkoxy," "alkoxy carbonyl," "carbamate," "sulfanyl," "halogen," trihalomethyl, cyano, hydroxy, mercapto, nitro, and the like. The term "pharmaceutically acceptable salts or complexes" refers to salts or complexes of the below-specified compounds of the invention. Examples of such salts include, but are not restricted, to base addition salts formed by reaction of compounds of the invention with organic or inorganic bases such as hydroxide, carbonate, bicarbonate or the like, of a metal cation such as those selected in the group consisting of alkali metals (sodium, potassium or lithium), alkaline earth metals (e.g. calcium or magnesium), or with an organic primary, secondary or tertiary alkyl amine. Other examples of such salts include, but are not restricted, to acid addition salts formed by reaction of compounds of the invention with organic or inorganic acids such as hydrochloric acid, hydrobromic acid, sulphuric acid, para-toluene sulfonic acid, 2-naphtalene sulfonic acid, camphosulfonic acid, benzene sulfonic acid, oxalic acid or the like.

"Pharmaceutically active derivative" refers to any compound that upon administration to the recipient, is capable of providing directly or indirectly, the activity disclosed herein. The term "indirectly" also encompasses prodrugs which may be converted to the active form of the drug via endogenous enzymes or metabolism. The prodrug is a derivative of the compound according to the invention and presenting the described activity that has a chemically or metabolically decomposable group, and a compound that may be converted into a pharmaceutically active compound in vivo by solvolysis under physiological conditions. The invention further encompasses any tautomers of the compounds according to the invention.

### Compounds according to the invention

In one embodiment, the invention provides a compound of Formula (I) 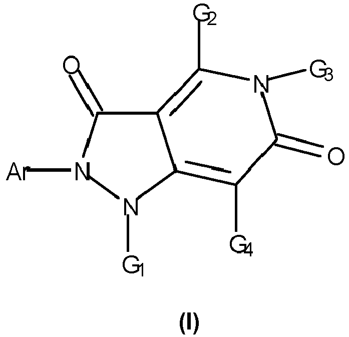 wherein **Ar** is optionally substituted phenyl such as phenyl optionally substituted by halogen such as chloro (e.g. 2-chlorophenyl); **G₁** and **G₄** are **H; G**₂ is selected from optionally substituted C₁-C₆ alkyl (e.g. methyl) and optionally substituted phenyl (such as phenyl optionally substituted by halogen such as 3-chlorophenyl, 4-chlorophenyl, 2-fluorophenyl, 3-fluorophenyl, 4-fluorophenyl, 4-chloro-2-fluorophenyl, 5-chloro-2-fluorophenyl, phenyl optionally substituted by amino or alkyl amino or alkoxy such as 3-dimethylaminophenyl, 2- tri-methyl amino phenyl, 3-methyl amino phenyl, 3-amino phenyl, 4-methoxy phenyl); **G₃** is selected from H, optionally substituted C₁-C₆ alkyl (e.g. methyl, C₁-C₆ alkyl substituted by alkoxy like methoxy ethyl such as 2-methoxyethyl), optionally substituted heteroaryl C₁-C₆ alkyl like optionally substituted pyridinyl C₁-C₆ alkyl (e.g. optionally substituted pyridinyl methyl like pyridinyl-2ylmethyl, pyridinyl-3ylmethyl, 6-methoxypyridin-3-yl methyl, 2-methoxypyridin-4-yl methyl) or optionally substituted pyrazinyl C₁-C₆ alkyl (e.g. pyrazinyl-2-ylmethyl) and optionally substituted alkoxy C₁-C₆ alkyl such as methoxy ethyl (e.g. 2 methoxyethyl) or G₂ and G₃ form together an optionally substituted 7-membered heterocycloalkyl ring comprising two nitrogen atoms, and where the two nitrogens are attached through a optionally substituted C₁-C₃ alkyl moiety, as well as tautomers, geometrical isomers, optically active forms and pharmaceutically acceptable salts thereof use in the prevention and/or treatment of PDE5 inhibitor non-responsive or poorly responsive erectile dysfunction.

In a particular embodiment, the invention provides a compound of Formula (I) wherein G₂ and G₃ form together an optionally substituted 7-membered heterocycloalkyl ring comprising two nitrogen atoms to form the following compound of Formula (I'): 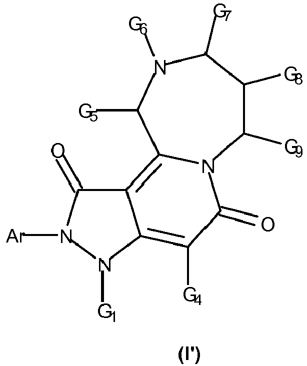 wherein **Ar, G₁** and **G₄** are as defined herein; **G₅**, **G₇** to **G₉** are **H; G₆** is selected from optionally substituted C₁-C₆ alkyl such as C₁-C₆ alkyl optionally substituted with optionally substituted phenyl (e.g. methyl optionally substituted with optionally substituted phenyl such as benzyl, methyl optionally substituted with phenyl substituted by halogen such as 2-chlorobenzyl, 3-chlorobenzyl, 4-chlorobenzyl, methyl optionally substituted with phenyl substituted by alkoxy such as 2-methoxybenzyl, 3-methoxybenzyl, 4-methoxybenzyl), optionally substituted aryl C₁-C₆ alkyl such as optionally substituted phenyl C₁-C₆ alkyl (e.g. benzyl, 2-chlorobenzyl, 3-chlorobenzyl, 4-chlorobenzyl, 2-methoxybenzyl, 3-methoxybenzyl, 4-methoxybenzyl) and optionally substituted heteroaryl C₁-C₆ alkyl such as optionally substituted pyridinyl C₁-C₆ alkyl (e.g. optionally substituted pyridinyl methyl like pyridinyl-2ylmethyl, pyridinyl-3ylmethyl) or optionally substituted furanyl C₁-C₆ alkyl (e.g. optionally substituted furanyl methyl like furan-3ylmethyl) for use in the prevention and/or treatment of PDE5 inhibitor non-responsive or poorly responsive erectile dysfunction.

In a particular embodiment, the invention provides a compound of Formula (I) for use according to the invention wherein **G**₂ is optionally substituted C₁-C₆ alkyl.

In another particular embodiment, the invention provides a compound of Formula (I) for use according to the invention wherein **G**₂ is optionally substituted phenyl.

In another particular embodiment, the invention provides a compound of Formula (I) for use according to the invention wherein **G₃** is optionally substituted C₁-C₆ alkyl.

In another particular embodiment, the invention provides a compound of Formula (I) for use according to the invention wherein **G₃** is optionally substituted heteroaryl C₁-C₆ alkyl like optionally substituted pyridinyl C₁-C₆ alkyl.

In another particular embodiment, the invention provides a compound of Formula (I) for use according to the invention wherein G₂ and G₃ form together an optionally substituted 7-membered heterocycloalkyl ring comprising two nitrogen atoms to form the following compound of Formula (I'), wherein **G₆** is optionally substituted C₁-C₆ alkyl.

In another the invention provides a compound of Formula (I) for use according to the invention wherein G₂ and G₃ form together an optionally substituted 7-membered heterocycloalkyl ring comprising two nitrogen atoms to form the following compound of Formula (I'), wherein **G₆** is optionally substituted aryl C₁-C₆ alkyl.

In another the invention provides a compound of Formula (I) for use according to the invention wherein G₂ and G₃ form together an optionally substituted 7-membered heterocycloalkyl ring comprising two nitrogen atoms to form the following compound of Formula (I'), wherein **G₆** is optionally substituted heteroaryl C₁-C₆ alkyl

In another particular embodiment, is provided a compound according to the invention selected from the following group:
2-(2-chlorophenyl)-4-methyl-5-(pyridin-2-ylmethyl)-1H-pyrazolo[4,3-c]pyridine-3,6(2H,5H)-dione;
2-(2-chlorophenyl)-4-(3-chlorophenyl)-5-(2-methoxyethyl)-1H-pyrazolo[4,3-c] pyridine-3,6(2H,5H)-dione;
2-(2-chlorophenyl)-4-(4-chlorophenyl)-5-(2-methoxyethyl)-1H-pyrazolo[4,3-c] pyridine-3,6(2H,5H)-dione;
2-(2-chlorophenyl)-4-(2-fluorophenyl)-5-methyl-1H-pyrazolo[4,3-c]pyridine-3,6 (2H,5H)-dione;
2-(2-chlorophenyl)-4-[3-(dimethylamino)phenyl]-5-methyl-1H-pyrazolo[4,3-c]pyridine-3,6(2H,5H)-dione;
4-(4-chloro-2-fluorophenyl)-2-(2-chlorophenyl)-5-(pyridin-3-ylmethyl)-1H-pyrazolo [4,3-c]pyridine-3,6(2H,5H)-dione;
2-(2-chlorophenyl)-4-(2-fluorophenyl)-5-(pyridin-3-ylmethyl)-1H-pyrazolo[4,3-c] pyridine-3,6(2H,5H)-dione;
4-(5-chloro-2-fluorophenyl)-2-(2-chlorophenyl)-5-(pyridin-4-ylmethyl)-1H-pyrazolo [4,3-c]pyridine-3,6(2H,5H)-dione;
4-(5-chloro-2-fluorophenyl)-2-(2-chlorophenyl)-5-(pyridin-3-ylmethyl)-1H-pyrazolo [4,3-c]pyridine-3,6(2H,5H)-dione;
10-benzyl-2-(2-chlorophenyl)-2,3,8,9,10,11-hexahydro-1H-pyrazolo[4',3':3,4]pyrido [1,2-a][1,4]diazepine-1,5(7H)-dione;
2-(2-chlorophenyl)-4-(4-chlorophenyl)-5-(pyrazin-2-ylmethyl)-1H-pyrazolo[4,3-c] pyridine-3,6(2H,5H)-dione;
10-(3-chlorobenzyl)-2-(2-chlorophenyl)-2,3,8,9,10,11-hexahydro-1H-pyrazolo [4',3' :3,4]pyrido[1,2-a][1,4]diazepine-1,5(7H)-dione;
10-(4-chlorobenzyl)-2-(2-chlorophenyl)-2,3,8,9,10,11-hexahydro-1H-pyrazolo [4',3' :3,4]pyrido[1,2-a][1,4]diazepine-1,5(7H)-dione;
10-(2-chlorobenzyl)-2-(2-chlorophenyl)-2,3,8,9,10,11-hexahydro-1H-pyrazolo [4',3' :3,4]pyrido[1,2-a][1,4]diazepine-1,5(7H)-dione;
2-(2-chlorophenyl)-10-(2-methoxybenzyl)-2,3,8,9,10,11-hexahydro-1H-pyrazolo [4',3' :3,4]pyrido[1,2-a][1,4]diazepine-1,5(7H)-dione;
2-(2-chlorophenyl)-10-(3-methoxybenzyl)-2,3,8,9,10,11-hexahydro-1H-pyrazolo [4',3' :3,4]pyrido[1,2-a][1,4]diazepine-1,5(7H)-dione;
2-(2-chlorophenyl)-10-(pyridin-2-ylmethyl)-2,3,8,9,10,11-hexahydro-1H-pyrazolo [4',3' :3,4]pyrido[1,2-a][1,4]diazepine-1,5(7H)-dione;
2-(2-chlorophenyl)-10-(4-methoxybenzyl)-2,3,8,9,10,11-hexahydro-1H-pyrazolo [4',3' :3,4]pyrido[1,2-a][1,4]diazepine-1,5(7H)-dione;
2-(2-chlorophenyl)-10-(furan-3-ylmethyl)-2,3,8,9,10,11-hexahydro-1H-pyrazolo [4',3' :3,4]pyrido[1,2-a][1,4]diazepine-1,5(7H)-dione;
2-(2-chlorophenyl)-5-[(6-methoxypyridin-3-yl)methyl]-4-methyl-1H-pyrazolo[4,3-c] pyridine-3,6(2H,5H)-dione;
2-(2-chlorophenyl)-5-[(2-methoxypyridin-4-yl)methyl]-4-methyl-1H-pyrazolo[4,3-c] pyridine-3,6(2H,5H)-dione;
2-(2-chlorophenyl)-4-(4-methoxyphenyl)-5-(pyrazin-2-ylmethyl)-1H-pyrazolo[4,3-c] pyridine-3,6(2H,5H)-dione;
2-(2-chlorophenyl)-10-(pyridin-3-ylmethyl)-2,3,8,9,10,11-hexahydro-1H-pyrazolo [4',3':3,4]pyrido[1,2-a][1,4]diazepine-1,5(7H)-dione;
2-(2-chlorophenyl)-5-methyl-4-[3-(methylamino)phenyl]-1H-pyrazolo[4,3-c]pyridine-3,6(2H,5H)-dione;
4-(3 -aminophenyl)-2-(2-chlorophenyl)-5-methyl-1H-pyrazolo [4, 3-c]pyridine-3 , 6 (2H,5H)-dione;
3-[2-(2-chlorophenyl)-5-methyl-3,6-dioxo-2,3,5,6-tetrahydro-1H-pyrazolo[4,3-c] pyridin-4-yl]-N,N,N-trimethylanilinium;
2-(2-chlorophenyl)-4-(4-fluorophenyl)-5-(2-methoxyethyl)-1H-pyrazolo[4,3-c]pyridine-3,6(2H,5H)-dione;
2-(2-chlorophenyl)-4-(4-fluorophenyl)-5-methyl-1H-pyrazolo[4,3-c]pyridine-3,6 (2H,5H)-dione;
2-(2-chlorophenyl)-4-(3-fluorophenyl)-5-(2-methoxyethyl)-1H-pyrazolo[4,3-c]pyridine-3,6(2H,5H)-dione; and
2-(2-chlorophenyl)-4-(3-fluorophenyl)-5-methyl-1H-pyrazolo[4,3-c]pyridine-3,6 (2H,5H)-dione.

In another particular embodiment, is provided compound of Formula (I) for use according to the invention, wherein the compound is 2-(2-chlorophenyl)-4-[3-(dimethylamino)phenyl]-5-methyl-1H-pyrazolo[4,3-c] pyridine-3,6(2H,5H)-dione.

In another particular embodiment, is provided compound of Formula (I) for use according to the invention, wherein the compound is 2-(2-chlorophenyl)-4-[3-(dimethylamino)phenyl]-5-methyl-1H-pyrazolo[4,3-c] pyridine-3,6(2H,5H)-dione and wherein the compound of Formula (I) is to be administered in combination with a PDE5 inhibitor.

In another particular embodiment, is provided compound of Formula (I) for use according to the invention, wherein the compound is 2-(2-chlorophenyl)-4-[3-(dimethylamino)phenyl]-5-methyl-1H-pyrazolo[4,3-c] pyridine-3,6(2H,5H)-dione and wherein the compound of Formula (I) is to be administered in combination with sildenafil.

In another particular embodiment, is provided a compound according to the invention selected from the following group:
2-(2-chlorophenyl)-4-(3-chlorophenyl)-5-(2-methoxyethyl)-1H-pyrazolo[4,3-c] pyridine-3,6(2H,5H)-dione;
2-(2-chlorophenyl)-5-[(6-methoxypyridin-3-yl)methyl]-4-methyl-1H-pyrazolo[4,3-c] pyridine-3,6(2H,5H)-dione;
4-(3-aminophenyl)-2-(2-chlorophenyl)-5-methyl-1H-pyrazolo[4,3-c]pyridine-3,6 (2H,5H)-dione;
3-[2-(2-chlorophenyl)-5-methyl-3,6-dioxo-2,3,5,6-tetrahydro-1H-pyrazolo[4,3-c] pyridin-4-yl]-N,N,N-trimethylanilinium;
2-(2-chlorophenyl)-4-(4-fluorophenyl)-5-(2-methoxyethyl)-1H-pyrazolo[4,3-c]pyridine-3,6(2H,5H)-dione;
2-(2-chlorophenyl)-4-(4-fluorophenyl)-5-methyl-1H-pyrazolo[4,3-c]pyridine-3,6 (2H,5H)-dione;
2-(2-chlorophenyl)-4-(3-fluorophenyl)-5-(2-methoxyethyl)-1H-pyrazolo[4,3-c]pyridine-3,6(2H,5H)-dione; and
2-(2-chlorophenyl)-4-(3-fluorophenyl)-5-methyl-1H-pyrazolo[4, 3-c]pyridine-3 , 6 (2H,5H)-dione.

In another particular embodiment, is provided a compound of Formula (I) according of the invention for use as a medicament.

In another particular embodiment, compounds for use according to the invention are for use in restoring or increasing responsiveness to PDE5 inhibitor treatment, in particular restoring or increasing responsiveness of endothelial tissues of *corpus cavernosum* to a PDE5 inhibitor in a patient.

In another particular embodiment, compounds for use according to the invention are to be administered in combination (e.g. prior or concomitantly) to a PDE5 inhibitor.

### Compositions

The invention provides pharmaceutical or therapeutic agents as compositions and methods for treating a patient, preferably a mammalian patient, and most preferably a human patient who is suffering from PDE5 inhibitor non-responsive or poorly responsive erectile dysfunction.

Pharmaceutical compositions of the invention can contain one or more compound of Formula (I) in any form described herein. Compositions of this invention may further comprise one or more pharmaceutically acceptable additional ingredient(s), such as alum, solubilizers, stabilizers, antimicrobial agents, buffers, coloring agents, flavoring agents, adjuvants, and the like.

The compounds of the invention, together with a conventionally employed adjuvant, carrier, diluent or excipient may be placed into the form of pharmaceutical compositions and unit dosages thereof, and in such form may be employed as solids, such as powder in sachets, tablets or filled capsules, or liquids such as solutions, suspensions, emulsions, elixirs, nasal spray, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. Compositions according to the invention are preferably oral, sublingual, nasal and subcutaneous.

Compositions of this invention may also be liquid formulations, including, but not limited to, aqueous or oily suspensions, solutions, emulsions, syrups, spray and elixirs. Liquid forms suitable for oral administration may include a suitable aqueous or nonaqueous vehicle with buffers, suspending and dispensing agents, colorants, flavors and the like. The compositions may also be formulated as a dry product for reconstitution with water or other suitable vehicle before use. Such liquid preparations may contain additives, including, but not limited to, suspending agents, emulsifying agents, nonaqueous vehicles and preservatives. Suspending agents include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Non aqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in The Science and Practice of Pharmacy (Remington: The Science & Practice of Pharmacy), 22nd Edition, 2012, Lloyd, Ed. Allen, Pharmaceutical Press*,* which is incorporated herein by reference.

Solid compositions of this invention may be in the form of powder in sachets, tablets or lozenges formulated in a conventional manner. For example, sachets, tablets and capsules for oral or sublingual administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Injectable compositions are typically based upon injectable sterile saline or phosphate-buffered saline or other injectable carriers known in the art.

Compositions of this invention may also be formulated for parenteral administration, including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

The compounds of this invention can also be administered in sustained release forms or from sustained release drug delivery systems. A description of representative sustained release materials can also be found in the incorporated materials in *Remington's Pharmaceutical Sciences.*

### Mode of administration

Compositions of this invention may be administered in any manner, including, but not limited to, orally, parenterally, sublingually, via buccal administration, nasally, , or combinations thereof. Parenteral administration includes, but is not limited to subcutaneous and intramuscular. The compositions of this invention may also be administered in the form of an implant, which allows slow release of the compositions as well as a slow controlled i.v. infusion. In a particular embodiment, one or more compound of Formula (I) is administered orally.

The dosage administered, as single or multiple doses, to an individual will vary depending upon a variety of factors, including pharmacokinetic properties, patient conditions and characteristics (age, body weight, health, body size), extent of symptoms, frequency of treatment and the effect desired.

### Combination

According to one embodiment of the invention, a compound of Formula (I) according to the invention and pharmaceutical formulations thereof is to be administered in combination with a PDE5 inhibitor, in particular sildenafil or sildenafil citrate.

The invention encompasses the administration of a compound of Formula (I) or a pharmaceutical formulation thereof, wherein a compound of Formula (I) or a pharmaceutical formulation thereof is administered to an individual prior to, or simultaneously with a PDE5 inhibitor, for example or concomitantly through the same formulation.

According to a particular aspect of the invention, compound of Formula (I) according to the invention and pharmaceutical formulations thereof is to be administered chronically (e.g. daily or weekly) for at least one week prior to the administration of a PDE5 inhibitor.

According to another particular aspect of the invention, compound of Formula (I) according to the invention and pharmaceutical formulations thereof is to be administered concomitantly with the PDE5 inhibitor.

According to another particular aspect of the invention, the PDE5 inhibitors can be administered in combination with other therapeutic regimens or co-agents useful in the treatment of erectile dysfunction (e.g. multiple drug regimens), in a therapeutically effective amount.

Compounds of Formula (I) or the pharmaceutical formulations thereof that are administered simultaneously with said PDE5 inhibitors can be administered in or within the same or different composition(s) and by the same or different route(s) of administration.

### Patients

In one embodiment, patients according to the invention are patients suffering from PDE5 inhibitor non-responsive or poorly responsive erectile dysfunction.

In a particular embodiment, patients according to the invention are type-2 diabetes mellitus patients suffering from PDE5 inhibitor non-responsive or poorly responsive erectile dysfunction.

In another particular embodiment, patients according to the invention are radical retropubic prostatectomy (RRP) patients suffering from PDE5 inhibitor non-responsive or poorly responsive erectile dysfunction.

In another particular embodiment, patients according to the invention are patients suffering from penile fibrosis and PDE5 inhibitor non-responsive or poorly responsive erectile dysfunction.

In another particular embodiment, patients according to the invention are patients suffering from severe vascular disease and PDE5 inhibitor non-responsive or poorly responsive erectile dysfunction.

In another particular embodiment, patients according to the invention are depressive patients presenting a PDE5 inhibitor non-responsive or poorly responsive erectile dysfunction.

In another particular embodiment, patients according to the invention are patients with erectile dysfunction who are at risk of developing resistance or partial resistance to PDE5 inhibitor due to another concomitant treatment.

### Use according to the invention

In another embodiment, the invention provides compounds of Formula (I) as well as tautomers, geometrical isomers, optically active forms and pharmaceutically acceptable salts thereof for the preparation of a pharmaceutical composition for the treatment or prophylaxis of PDE5 inhibitor non-responsive or poorly responsive erectile dysfunction.

In a particular embodiment, the invention provides compounds of Formula (I) for the preparation of a pharmaceutical composition for the treatment or prophylaxis of PDE5 inhibitor non-responsive or poorly responsive erectile dysfunction.

In another embodiment, the invention provides compounds of Formula (I) or pharmaceutical compositions thereof for the treatment or prophylaxis of PDE5 inhibitor non-responsive or poorly responsive erectile dysfunction.

In another embodiment, the invention provides compounds of Formula (I) or pharmaceutical compositions for the restoration of or increase in responsiveness to treatment with PDE5 inhibitors.

In another embodiment, the invention provides a method for treating a patient suffering from PDE5 inhibitor non-responsive or poorly responsive erectile dysfunction, the method comprises administering an effective amount of a compound of Formula (I) or a pharmaceutical formulation thereof in a patient in need thereof.

In another embodiment, the invention provides a method for restoring or increasing responsiveness to PDE5 inhibitor treatment, in particular restoring or increasing responsiveness of endothelial tissues of *corpus cavernosum* to a PDE5 inhibitor in a patient, the method comprises administering an effective amount a compound of Formula (I) or a pharmaceutical formulation thereof in a patient in need thereof.

In another embodiment, the invention provides a pharmaceutical composition containing at least one compound of Formula (I) according to the invention and a pharmaceutically acceptable carrier, diluent or excipient thereof.

In another embodiment, the invention provides a pharmaceutical composition combining at least one compound of Formula (I) according to the invention with at least one PDE5 inhibitor and a pharmaceutically acceptable carrier, diluent or excipient thereof.

In a particular embodiment, the compounds, pharmaceutical formulation and methods of the invention are contemplated for use in the prevention and/or treatment of PDE5 inhibitor non-responsive or poorly responsive erectile dysfunction.

In a particular embodiment, the invention provides a compound of Formula (I) according to the invention or pharmaceutical formulations thereof or a use thereof and methods according to the invention wherein the compound of Formula (I) is to be administered in combination with a PDE5 inhibitor.

In a further particular embodiment, the invention provides, a compound of Formula (I) according to the invention or pharmaceutical formulations thereof or a use thereof and methods according to the invention wherein the compound of Formula (I) is to be administered in combination with a PDE5 inhibitor, using either by a single formulation or mode of administration or two distinct formulations and modes/routes of administration.

The compounds of invention have been named according the IUPAC standards used in the program ACD/Name (product version 10.01).

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

The invention having been described, the following examples are presented by way of illustration, and not limitation.

### EXAMPLES

The efficacy of compounds of Formula (I) for restoring or increasing responsiveness to PDE5 inhibitor treatment is supported by the following experiments.

### The following abbreviations refer respectively to the definitions below:

**AUC** (area under the curve), **HPβCD** (hydroxypropyl-beta-cyclodextrin), **ICP** (intra-cavemosum pressure), **IV** (intraveneously), **MAP** (mean arterial pressure).

### Example 1: Erectile function in a model of type II diabetes mellitus associated erectile dysfunction with impaired sildenafil response

The efficacy of compounds of Formula (I) for restoring or increasing responsiveness to treatment with a PDE5 inhibitor (sildenafil) is assessed in a rat model of type II diabetes mellitus associated erectile dysfunction in Goto-Kakizaki (GK) rats. Those rats show the main features of the metabolic, hormonal and vascular disorders usually described in T2D patients (Portha et al., 2001, Diabetes 50 (Suppl. 1): S89-S93*;* Portha et al., 1991, Diabetes 40, 486-491)*.*

Adult male Wistar and age-matched Goto-Kakizaki (GK) rats (obtained by repeated inbreeding of non-diabetic Wistar rats selected at the upper limit or normal distribution if glucose tolerance as described in Goto et al., 1975, Proc. Jpn Acad, 51, 80-5 are housed at least 7 days prior to the beginning of the experiments with free access to standard chow and water and maintained on an inversed 12 h dark/light cycle (10:00/22:00).

At 15 weeks of age, treatment period is started; n=24 GK rats (Groups 4 & 5) receive a dose of compound 5 (30 mg/kg) while n=24 GK (Groups 1 and 3) rats and n=12 Wistar (Group 2) rats receive an equivalent volume of vehicle (25% HPβCD) of compound 5 by daily oral gavage for 28 days.

The last day of treatment (day 28^{th} after starting treatment administration), urine is collected for a period of 24 h (after a 24-h period of acclimation to the metabolic cage) for 36 rats (Groups 1, 2 & 4), frozen and stored at -80°C for further analyses.

On day 29^{th} after starting treatment administration, the rats (19 weeks of age) undergo an evaluation of their erectile response measured after electrical stimulation (ES) of the cavernous nerve (CN) by monitoring intracavernous pressure (ICP). For this purpose, rats are anesthetized (urethane 1.2 mg/kg) after a 3-hour fasting period (necessary for plasma glucose dosage), their temperature maintained at 37°C using a homeothermic blanket. The carotid artery was catheterized to record blood pressure (BP) via a pressure transducer (Elcomatic 750, Glasgow, UK). Both jugular veins were also catheterised to allow intravenous injections. CN is surgically exposed at the lateral aspect of the prostate and mounted on a bipolar platinum electrode connected to an electrical stimulator (AMS 2100, Phymep, France).

On day 29^{th} animals receiving chronic administration of GKT137831 or vehicle (groups 4 & 5) did not receive a further administration of test agent. As a consequence, there are 16 hours between animals receiving their final administration of compound 5 or vehicle and initiation of evaluation of their erectile function. Rats were anesthetized and then received a 1st intravenous injection (iv) 25%HPβCD, the vehicle of compound 5, 60 minutes prior to the evaluation period of treatment effect. All rats also received a 2^{nd} iv injection of either sildenafil (Sequoia research, Pangbourne, UK) 0.3mg/kg (groups 3 & 5) or normal saline, the vehicle of sildenafil, (groups 1, 2, 4) 56 minutes after the first injection.

At T=-15 minutes, the continuous recording of BP and ICP started. When diastolic BP was inferior to 40 mmHg during the first 5 minutes of recording, rats were not considered further for the experiment and were discarded. Two stimulations of the CN at 15Hz, 1 ms for 45 s were performed to elicit an increase of ICP to certify the correct implantation of the catheter and to assess baseline erectile response prior treatment

At T=0 minute, 25% HPβCD was injected intravenously.

At T+4 minutes and T+30 minutes, the CN was stimulated at 10Hz, 1ms for 45s.At T+56 minutes, sildenafil (0.3mg/kg) or normal saline was intravenously injected.

At T+ 60 minutes, and at 3 minutes intervals thereafter, the CN was stimulated at 6V, 1 ms for 45 s by different stimulation frequencies (10 Hz then 0, 2.5, 5, 7.5, 12.5 and 15 Hz) in a randomized manner in order to assess the erectile responses. These different electrical stimulations were repeated twice in view of establishing a frequency-response curve for each animal.

At T+ 120 minutes, the recording ended.

Mean arterial pressure (MAP) and the amplitude of the erectile responses elicited by each ES CN is quantified for each rat and averaged for each experimental group by calculating:
- ICP (mmHg) / MAP (mmHg) x 100 with ΔICP being the difference between ICP in the flaccid state, i.e. before stimulation and ICP during the plateau phase of the erectile response, and MAP, the mean arterial pressure during the plateau phase.
- AUCₜₒₜ / MAP with AUCₜₒₜ, the area under the curve during the entire erectile response, measured from the beginning of the electrical stimulation until the end of the erectile response and determined using the ICP level in the flaccid state before the onset of the stimulation.
- AUC₄₅ / MAP with AUC₄₅, the area under the curve during the first 45 seconds after the beginning of the electrical stimulation of the cavernous nerve.

ICP increase and AUC are normalized with MAP to account for the close influence of the systemic blood pressure in the amplitude of ICP increase during the plateau phase of the erectile response.

At the end of this experimental period, a blood sample for n=24 GK rats (Group 1 & 4) and n=12 wistar rats (Group 2) is collected and stored at +4°C for a period of 24 h maximum for plasma glucose dosage.

For erectile function evaluation, comparisons of frequency-response curves are performed with a two-way ANOVA statistical analysis test followed by a Bonferroni's post-test (Miller, 1981, Simultaneous statistical inference, 2nd ed. Springer Verlag, pages 6-8). In case of significant interaction between the two factors (i.e. frequency of ES CN and experimental group), the difference between groups of rats are examined by the modified Student's t-test with the Bonferroni's adjustment for multiple comparisons. Statistical analysis is performed with GraphPad Prism® 5.02 software. P values < 0.05 will be considered significant.

The penis of these 36 rats are harvested and the *corpora cavernosa* (CC) dissected, snap frozen and stored at -80°C.The kidneys of these same rats will also be harvested, snap frozen and stored at -80°C. All rats are then euthanized by an overdose of urethane

All results will be presented as mean ± SEM. Grubbs test will be used for the detection and exclusion of outliers. This statistical method allowed determining when a value was unlikely to have come from the same Gaussian population as the other values in the group.

The administration of compound 5 alone at 30mg/kg once daily for 4 weeks improved the erectile responses to ES CN in diabetic GK rats, thus eliciting statistically greater overall erectile responses in terms AUC₄₅/MAP (p≤0.05, figure 1A) compared to those observed in diabetic GK rats treated with vehicle. In contrast, no statistical difference was observed for AUCₜₒₜ/MAP (p=0.147, figure 1B). Similarly to sildenafil, the erectile responses following chronic administration of compound 5 at 30mg/kg/day were significantly lower than those measured in healthy Wistar rats (Figure 1A).

The pro-erectile facilitator effects elicited by each compound alone, i.e. chronic compound 5 at 30mg/kg/day and acute sildenafil iv single administration at 0.3mg/kg, in diabetic GK rats were further increased when both treatment strategies were combined as assessed AUC₄₅/MAP (p≤0.0001 vs. GK rats, 2-way ANOVA, Figure 1A) and AUCₜₒₜ/MAP (p≤0.0001 vs. GK rats, 2-way ANOVA with interaction, figure 1B). In fact, the increase in terms of AUCₜₒₜ/MAP was statistically significant when compared to each compound administered alone (AUCₜₒₜ/MAP: p≤0.0001, 2-way ANOVA with interaction and p≤0.05, 2-way ANOVA when comparing the combined effect to acute sildenafil and chronic compound 5, Figure 1B).

The results indicated that acute sildenafil 0.3 mg/kg combined with chronic compound 5 30mg/kg/day in diabetic GK rats restored erectile responses to ES CN in terms of AUCₜₒₜ/MAP (Figure 1B). Indeed, this AUCₜₒₜ/MAP following ES CN stimulation in diabetic GK rats treated with the combination therapy, was not significantly different from AUCₜₒₜ/MAP in control healthy Wistar rats (Figure 1B).

Therefore, 4 weeks of once daily oral administration of a compound of Formula (I) at a dose of 30 mg/kg was, when used in combination with Sildenafil, were able to restore wild-type erectile responses to diabetic rats.

All those data support the effects of compounds of the invention in the reversal of PDE5 (Sildenafil) insensitivity in diabetic rats and the restoration of the response of their *corpora cavernosa* close to levels statistically indistinguishable from wild-type when used in combination with Sildenafil.

### Example 2: Compounds of Formula (I)

The following compounds listed in Table 2 below are examples of compounds of Formula (I) according to the invention that could be used according to the invention:

**Table 2**

| **Compound** | **Structure** | **Name** |
|---|---|---|
| **1** | 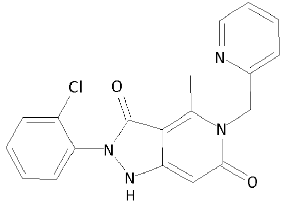 | 2-(2-chlorophenyl)-4-methyl-5-(pyridin-2-ylmethyl)-1H-pyrazolo [4,3-c]pyridine-3,6(2H,5H)-dione |
| **2** | 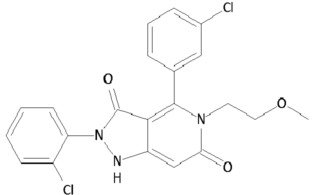 | 2-(2-chlorophenyl)-4-(3-chloro phenyl)-5-(2-methoxyethyl)-1H-pyrazolo[4,3-c]pyridine-3,6(2H,5H)-dione |
| **3** | 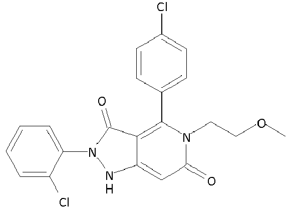 | 2-(2-chlorophenyl)-4-(4-chlorophenyl)-5-(2-methoxyethyl)-1H-pyrazolo[4,3-c] pyridine-3,6(2H,5H)-dione |
| **4** | 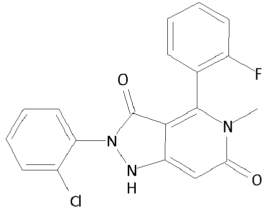 | 2-(2-chlorophenyl)-4-(2-fluorophenyl)-5-methyl-1H-pyrazolo[4,3-c]pyridine-3,6 (2H,5H)-dione |
| **5** | 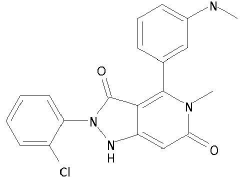 | 2-(2-chlorophenyl)-4-[3-(dimethyl amino)phenyl]-5-methyl-1H-pyrazolo[4,3-c] pyridine-3,6(2H,5H)-dione |
| **6** | 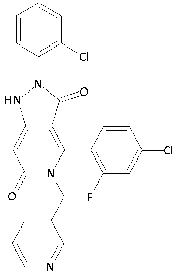 | 4-(4-chloro-2-fluorophenyl)-2-(2-chloro phenyl)-5-(pyridin-3-ylmethyl)-1H-pyrazolo[4,3-c]pyridine-3,6(2H,5H)-dione |
| **7** | 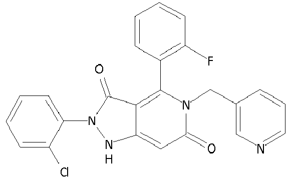 | 2-(2-chlorophenyl)-4-(2-fluorophenyl)-5-(pyridin-3-ylmethyl)-1H-pyrazolo[4,3-c]pyridine-3,6(2H,5H)-dione |
| **8** | 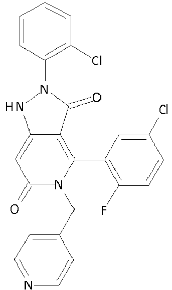 | 4-(5-chloro-2-fluorophenyl)-2-(2-chloro phenyl)-5-(pyridin-4-ylmethyl)-1H-pyrazolo[4,3-c]pyridine-3,6(2H,5H)-dione |
| **9** | 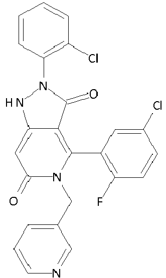 | 4-(5-chloro-2-fluorophenyl)-2-(2-chloro phenyl)-5-(pyridin-3-ylmethyl)-1H-pyrazolo[4,3-c]pyridine-3,6(2H,5H)-dione |
| **10** | 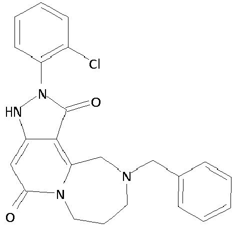 | 10-benzyl-2-(2-chlorophenyl)-2,3,8, 9,10,11-hexahydro-1H-pyrazolo [4',3':3,4]pyrido[1,2-a][1,4]diazepine-1,5(7H)-dione |
| **11** | 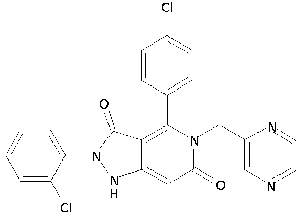 | 2-(2-chlorophenyl)-4-(4-chlorophenyl)-5-(pyrazin-2-ylmethyl)-1H-pyrazolo[4,3-c] pyridine-3,6(2H,5H)-dione |
| **12** | 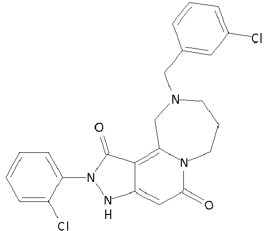 | 10-(3-chlorobenzyl)-2-(2-chlorophenyl)-2,3,8,9,10,11-hexahydro-1H-pyrazolo [4',3':3,4]pyrido[1,2-a][1,4]diazepine-1,5(7H)-dione |
| **13** | 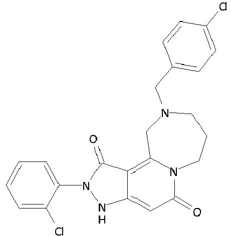 | 10-(4-chlorobenzyl)-2-(2-chlorophenyl)-2,3,8,9,10,11-hexahydro-1H-pyrazolo [4',3':3,4]pyrido[1,2-a][1,4]diazepine-1,5(7H)-dione |
| **14** | 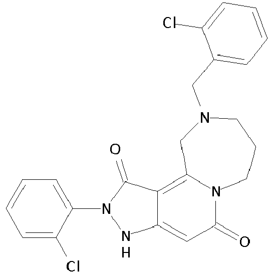 | 10-(2-chlorobenzyl)-2-(2-chlorophenyl)-2,3,8,9,10,11-hexahydro-1H-pyrazolo [4',3':3,4]pyrido[1,2-a][1,4]diazepine-1,5(7H)-dione |
| **15** | 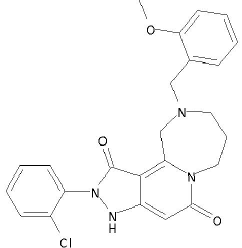 | 2-(2-chlorophenyl)-10-(3-methoxybenzyl)-2,3,8,9,10,11-hexahydro-1H-pyrazolo [4',3':3,4]pyrido[1,2-a][1,4]diazepine-1,5(7H)-dione |
| **16** | 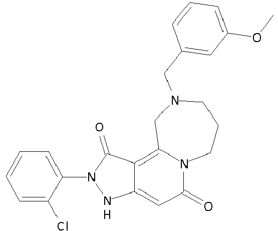 | 2-(2-chlorophenyl)-10-(3-methoxybenzyl)-2,3,8,9,10,11-hexahydro-1H-pyrazolo [4',3':3,4]pyrido[1,2-a][1,4]diazepine-1,5(7H)-dione |
| **17** | 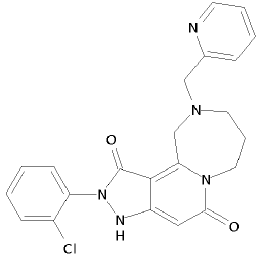 | 2-(2-chlorophenyl)-10-(pyridin-2-yl methyl)-2,3,8,9,10,11-hexahydro-1H-pyrazolo[4',3':3,4]pyrido[1,2-a] [1,4]diazepine-1,5(7H)-dione |
| **18** | 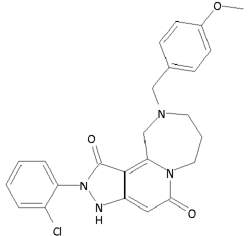 | 2-(2-chlorophenyl)-10-(4-methoxybenzyl)-2,3,8,9,10,11-hexahydro-1H-pyrazolo [4',3':3,4]pyrido[1,2-a][1,4]diazepine-1,5(7H)-dione |
| **19** | 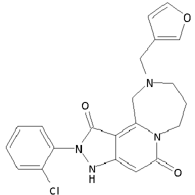 | 2-(2-chlorophenyl)-10-(furan-3-ylmethyl)-2,3,8,9,10,11-hexahydro-1H-pyrazolo[4',3':3,4]pyrido[1,2-a] [1,4]diazepine-1,5(7H)-dione |
| **20** | 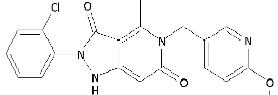 | 2-(2-chlorophenyl)-5-[(6-methoxypyridin-3-yl)methyl]-4-methyl-1H-pyrazolo[4,3-c]pyridine-3,6(2H,5H)-dione |
| **21** | 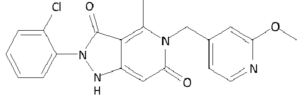 | 2-(2-chlorophenyl)-5-[(2-methoxypyridin-4-yl)methyl]-4-methyl-1H-pyrazolo[4,3-c]pyridine-3,6(2H,5H)-dione |
| **22** | 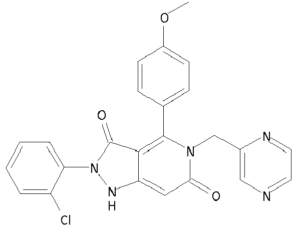 | 2-(2-chlorophenyl)-4-(4-methoxyphenyl)-5-(pyrazin-2-ylmethyl)-1H-pyrazolo[4,3-c]pyridine-3,6(2H,5H)-dione |
| **23** | 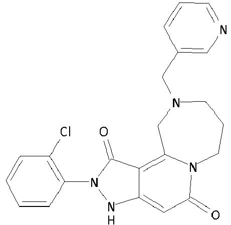 | 2-(2-chlorophenyl)-10-(pyridin-3-yl methyl)-2,3,8,9,10,11-hexahydro-1H-pyrazolo[4',3':3,4]pyrido[1,2-a] [1,4]diazepine-1,5(7H)-dione |
| **24** | 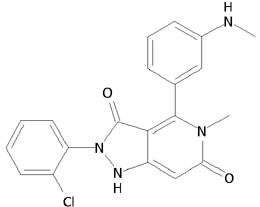 | 2-(2-chlorophenyl)-5-methyl-4-[3-(methyl amino)phenyl]-1H-pyrazolo[4,3-c] pyridine-3,6(2H,5H)-dione |
| **25** | 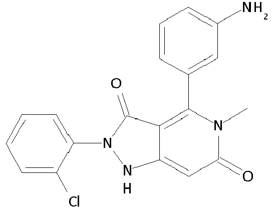 | 4-(3-aminophenyl)-2-(2-chlorophenyl)-5-methyl-1H-pyrazolo[4,3-c]pyridine-3,6 (2H,5H)-dione |
| **26** | 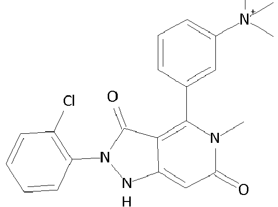 | 3-[2-(2-chlorophenyl)-5-methyl-3,6-dioxo-2,3,5,6-tetrahydro-1H-pyrazolo[4,3-c] pyridin-4-yl]-N,N,N-trimethylanilinium |
| **27** | 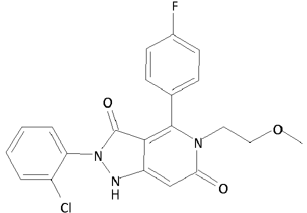 | 2-(2-chlorophenyl)-4-(4-fluorophenyl)-5-(2-methoxyethyl)-1H-pyrazolo[4,3-c] pyridine-3,6(2H,5H)-dione |
| **28** | 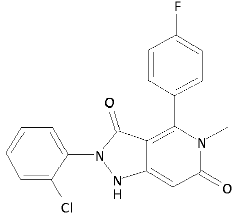 | 2-(2-chlorophenyl)-4-(4-fluorophenyl)-5-methyl-1H-pyrazolo[4,3-c]pyridine-3,6 (2H,5H)-dione |
| **29** | 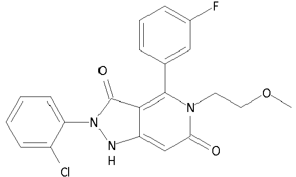 | 2-(2-chlorophenyl)-4-(3-fluorophenyl)-5-(2-methoxyethyl)-1H-pyrazolo[4,3-c] pyridine-3,6(2H,5H)-dione |
| **30** | 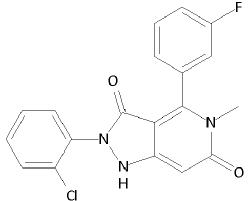 | 2-(2-chlorophenyl)-4-(3-fluorophenyl)-5-methyl-1H-pyrazolo[4,3-c]pyridine-3,6 (2H,5H)-dione |

### Synthesis of compounds of the invention

The compounds of the invention can be prepared from readily available starting materials for example using the methods detailed in WO 2008/113856; WO 2011/036651 and WO 2010/035221. It will be appreciated that where typical or preferred experimental conditions (i.e. reaction temperatures, time, moles of reagents, solvents etc.) are given, other experimental conditions can also be used unless otherwise stated. Optimum reaction conditions may vary with the particular reactants or solvents used, but such conditions can be determined by the person skilled in the art, using routine optimisation procedures. If synthetic method below is not applicable and/or necessary intermediates for the synthesis of compounds, suitable methods of preparation known by a person skilled in the art should be used. In general, the synthesis pathways for any individual compound will depend on the specific substituents of each molecule and upon the ready availability of intermediates necessary; again such factors being appreciated by those of ordinary skill in the art. For all the protection and deprotection methods, see Philip J. Kocienski, in "Protecting Groups", Georg Thieme Verlag Stuttgart, 2005 and Theodora W. Greene and Peter G. M. Wuts in "Protective Groups in Organic Synthesis", Wiley Interscience, 4th Edition 2006*.*

Compounds of this invention can be isolated in association with solvent molecules by crystallization from evaporation of an appropriate solvent.

## Claims

1. A compound of Formula (I): 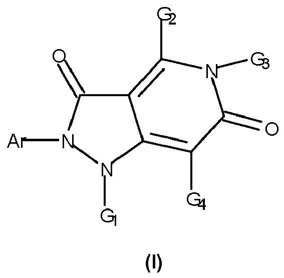 wherein **Ar** is optionally substituted phenyl; G₁ and G₄ are H; **G**₂ is selected from optionally substituted C₁-C₆ alkyl and optionally substituted phenyl; **G₃** is selected from H, optionally substituted C₁-C₆ alkyl, optionally substituted heteroaryl C₁-C₆ alkyl and optionally substituted alkoxy C₁-C₆ alkyl or G₂ and G₃ form together an optionally substituted 7-membered heterocycloalkyl ring comprising two nitrogen atoms and where the two nitrogens are attached through an optionally substituted C₁-C₃ alkyl moiety, as well as tautomers, geometrical isomers, optically active forms and pharmaceutically acceptable salts thereof for use in the prevention and/or treatment of PDE5 inhibitor non-responsive or poorly responsive erectile dysfunction.

2. A compound for use according to claim 1 wherein **G**₂ is optionally substituted C₁-C₆ alkyl.

3. A compound for use according to claim 1 wherein **G**₂ is optionally substituted phenyl.

4. A compound for use according to any one of claims 1 to 3 wherein **G₃** is optionally substituted C₁-C₆ alkyl.

5. A compound for use according to claim 1 wherein **G**₂ and **G₃** form together an optionally substituted 7-membered heterocycloalkyl ring comprising two nitrogen atoms to form the following compound of Formula (I'): 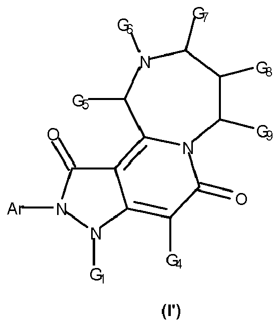 wherein **Ar, G₁** and **G₄** are as defined in any one of the preceding claims; **G₅**, **G₇** to **G₉** are H; **G₆** is selected from optionally substituted C₁-C₆ alkyl, optionally substituted aryl C₁-C₆ alkyl and optionally substituted heteroaryl C₁-C₆ alkyl.

6. A compound for use according to any one of claims 1 to 5 wherein the compound is to be administered in combination with a PDE5 inhibitor.

7. A compound for use according to any one of claims 1 to 6 wherein the PDE5 inhibitor is selected from sildenafil, sildenafil citrate, udenafil, avanafil, lodenafil, tadalafil, mirodenafil and vardenafil.

8. A compound for use according to claim 7 wherein the PDE5 inhibitor is selected from sildenafil and sildenafil citrate.

9. A compound of Formula (I) for use according to any one of claims 1 to 8 selected from the following group:
2-(2-chlorophenyl)-4-methyl-5-(pyridin-2-ylmethyl)-1H-pyrazolo[4,3-c]pyridine-3,6(2H,5H)-dione;
2-(2-chlorophenyl)-4-(3-chlorophenyl)-5-(2-methoxyethyl)-1H-pyrazolo[4,3-c] pyridine-3,6(2H,5H)-dione;
2-(2-chlorophenyl)-4-(4-chlorophenyl)-5-(2-methoxyethyl)-1H-pyrazolo[4,3-c] pyridine-3,6(2H,5H)-dione;
2-(2-chlorophenyl)-4-(2-fluorophenyl)-5-methyl-1H-pyrazolo[4,3-c]pyridine-3,6 (2H,5H)-dione;
2-(2-chlorophenyl)-4-[3-(dimethylamino)phenyl]-5-methyl-1H-pyrazolo[4,3-c] pyridine-3,6(2H,5H)-dione;
4-(4-chloro-2-fluorophenyl)-2-(2-chlorophenyl)-5-(pyridin-3-ylmethyl)-1H-pyrazolo[4,3-c]pyridine-3,6(2H,5H)-dione;
2-(2-chlorophenyl)-4-(2-fluorophenyl)-5-(pyridin-3-ylmethyl)-1H-pyrazolo[4,3-c] pyridine-3,6(2H,5H)-dione;
4-(5-chloro-2-fluorophenyl)-2-(2-chlorophenyl)-5-(pyridin-4-ylmethyl)-1H-pyrazolo[4,3-c]pyridine-3,6(2H,5H)-dione;
4-(5-chloro-2-fluorophenyl)-2-(2-chlorophenyl)-5-(pyridin-3-ylmethyl)-1H-pyrazolo[4,3-c]pyridine-3,6(2H,5H)-dione;
10-benzyl-2-(2-chlorophenyl)-2,3,8,9,10,11-hexahydro-1H-pyrazolo[4',3':3,4]pyrido [1,2-a][1,4]diazepine-1,5(7H)-dione;
2-(2-chlorophenyl)-4-(4-chlorophenyl)-5-(pyrazin-2-ylmethyl)-1H-pyrazolo[4,3-c] pyridine-3,6(2H,5H)-dione;
10-(3-chlorobenzyl)-2-(2-chlorophenyl)-2,3,8,9,10,11-hexahydro-1H-pyrazolo [4',3':3,4]pyrido[1,2-a][1,4]diazepine-1,5(7H)-dione;
10-(4-chlorobenzyl)-2-(2-chlorophenyl)-2,3,8,9,10,11-hexahydro-1H-pyrazolo [4',3':3,4]pyrido[1,2-a][1,4]diazepine-1,5(7H)-dione;
10-(2-chlorobenzyl)-2-(2-chlorophenyl)-2,3,8,9,10,11-hexahydro-1H-pyrazolo [4',3':3,4]pyrido[1,2-a][1,4]diazepine-1,5(7H)-dione;
2-(2-chlorophenyl)-10-(2-methoxybenzyl)-2,3,8,9,10,11-hexahydro-1H-pyrazolo [4',3':3,4]pyrido[1,2-a][1,4]diazepine-1,5(7H)-dione;
2-(2-chlorophenyl)-10-(3-methoxybenzyl)-2,3,8,9,10,11-hexahydro-1H-pyrazolo [4',3':3,4]pyrido[1,2-a][1,4]diazepine-1,5(7H)-dione;
2-(2-chlorophenyl)-10-(pyridin-2-ylmethyl)-2,3,8,9,10,11-hexahydro-1H-pyrazolo [4',3':3,4]pyrido[1,2-a][1,4]diazepine-1,5(7H)-dione;
2-(2-chlorophenyl)-10-(4-methoxybenzyl)-2,3,8,9,10,11-hexahydro-1H-pyrazolo [4',3':3,4]pyrido[1,2-a][1,4]diazepine-1,5(7H)-dione;
2-(2-chlorophenyl)-10-(furan-3-ylmethyl)-2,3,8,9,10,11-hexahydro-1H-pyrazolo [4',3':3,4]pyrido[1,2-a][1,4]diazepine-1,5(7H)-dione;
2-(2-chlorophenyl)-5-[(6-methoxypyridin-3-yl)methyl]-4-methyl-1H-pyrazolo[4,3-c]pyridine-3,6(2H,5H)-dione;
2-(2-chlorophenyl)-5-[(2-methoxypyridin-4-yl)methyl]-4-methyl-1H-pyrazolo[4,3-c]pyridine-3,6(2H,5H)-dione;
2-(2-chlorophenyl)-4-(4-methoxyphenyl)-5-(pyrazin-2-ylmethyl)-1H-pyrazolo[4,3-c]pyridine-3,6(2H,5H)-dione;
2-(2-chlorophenyl)-10-(pyridin-3-ylmethyl)-2,3,8,9,10,11-hexahydro-1H-pyrazolo [4',3':3,4]pyrido[1,2-a][1,4]diazepine-1,5(7H)-dione;
2-(2-chlorophenyl)-5-methyl-4-[3-(methylamino)phenyl]-1H-pyrazolo[4,3-c] pyridine-3,6(2H,5H)-dione;
4-(3-aminophenyl)-2-(2-chlorophenyl)-5-methyl-1H-pyrazolo[4,3-c]pyridine-3,6 (2H,5H)-dione;
3-[2-(2-chlorophenyl)-5-methyl-3,6-dioxo-2,3,5,6-tetrahydro-1H-pyrazolo[4,3-c] pyridin-4-yl]-N,N,N-trimethylanilinium;
2-(2-chlorophenyl)-4-(4-fluorophenyl)-5-(2-methoxyethyl)-1H-pyrazolo[4,3-c] pyridine-3,6(2H,5H)-dione;
2-(2-chlorophenyl)-4-(4-fluorophenyl)-5-methyl-1H-pyrazolo[4, 3-c]pyridine-3 , 6 (2H,5H)-dione;
2-(2-chlorophenyl)-4-(3-fluorophenyl)-5-(2-methoxyethyl)-1H-pyrazolo[4,3-c] pyridine-3,6(2H,5H)-dione; and
2-(2-chlorophenyl)-4-(3-fluorophenyl)-5-methyl-1H-pyrazolo[4,3-c]pyridine-3,6 (2H,5H)-dione.

10. A compound of Formula (I) for use according to any one of claims 1 to 8, wherein the compound is 2-(2-chlorophenyl)-4-[3-(dimethylamino)phenyl]-5-methyl-1H-pyrazolo[4,3-c]pyridine-3,6(2H,5H)-dione.

11. A compound according to Formula (I) selected from the following group:
2-(2-chlorophenyl)-4-(3-chlorophenyl)-5-(2-methoxyethyl)-1H-pyrazolo[4,3-c] pyridine-3,6(2H,5H)-dione;
2-(2-chlorophenyl)-5-[(6-methoxypyridin-3-yl)methyl]-4-methyl-1H-pyrazolo[4,3-c] pyridine-3,6(2H,5H)-dione; and
4-(3-aminophenyl)-2-(2-chlorophenyl)-5-methyl-1H-pyrazolo[4,3-c]pyridine-3,6 (2H,5H)-dione;
3-[2-(2-chlorophenyl)-5-methyl-3,6-dioxo-2,3,5,6-tetrahydro-1H-pyrazolo[4,3-c] pyridin-4-yl]-N,N,N-trimethylanilinium;
2-(2-chlorophenyl)-4-(4-fluorophenyl)-5-(2-methoxyethyl)-1H-pyrazolo[4,3-c]pyridine-3,6(2H,5H)-dione;
2-(2-chlorophenyl)-4-(4-fluorophenyl)-5-methyl-H-pyrazolo[4, 3-c]pyridine-3 , 6 (2H,5H)-dione;
2-(2-chlorophenyl)-4-(3-fluorophenyl)-5-(2-methoxyethyl)-1H-pyrazolo[4,3-c]pyridine-3,6(2H,5H)-dione; and
2-(2-chlorophenyl)-4-(3-fluorophenyl)-5-methyl-1H-pyrazolo[4,3-c]pyridine-3,6 (2H,5H)-dione.

12. A compound according to claim 11 for use as a medicament.

13. A pharmaceutical composition containing at least one compound according to claim 11, and a pharmaceutically acceptable carrier, diluent or excipient thereof.

14. A pharmaceutical composition containing at least one compound of Formula (I) combined with at least one PDE5 inhibitor, and at least one pharmaceutically acceptable carrier.

15. A pharmaceutical composition according to claim 14 wherein Formula (I) is 2-(2-chlorophenyl)-4-[3-(dimethylamino)phenyl]-5-methyl-1H-pyrazolo[4,3-c] pyridine-3,6(2H,5H)-dione.
